# EUROPEAN PATENT SPECIFICATION

(11) **EP 0 986 569 B1**
(45) Date of publication and mention of the grant of the patent: **24.09.2003**
(21) Application number: 98918330.6
(22) Date of filing: 17.04.1998
(51) Int. Cl.: C07H 15/24, C07H 1/00, C07H 17/00

(54) **PROCESS FOR PREPARING ETOPOSIDE**
VERFAHREN ZUR HERSTELLUNG VON ETOPOSID
PROCEDE DE PREPARATION D'ETOPOSIDE

(30) Priority: 02.06.1997 US 48292 P
(43) Date of publication of application: 22.03.2000
(73) Proprietor: Bristol-Myers Squibb Company, Wallingford, CT 06492-7660 (US)
(72) Inventor: SILVERBERG, Lee, Jonathan, Cherry Hill, NJ 08034 (US); VEMISHETTI, Purushotham, East Syracuse, NY 13057 (US)
(74) Representative: VOSSIUS & PARTNER
(86) International application number: US9807717
(87) International publication number: WO98055491

(56) References cited:
- WO-A-93/02094
- US-A- 4 757 138
- US-A- 5 036 055
- US-A- 5 206 350
- US-A- 5 459 248
- US-A- 5 688 926

## Description

### FIELD OF THE INVENTION

The present invention relates to a novel process for preparing the anticancer compound, etoposide, in the C-1"-β anomeric form, as well as a novel intermediate compound.

### BACKGROUND OF THE INVENTION

Etoposide, VP-16, a derivative of 4'-demethyl-4-epipodophyllotoxin, is sold by Bristol-Myers Squibb as Vepesid^{TM} and is widely used in clinical therapy of cancer. Etoposide was first reported by Kuhn *et al* of Sandoz Corporation in 1968¹. In a typical synthesis of etoposide (Scheme 1), 4'-demethyl-4-epipodophyllotoxin is protected at the phenol of the pendant aryl ring, then coupled with the anomeric hydroxyl group of a protected glucose derivative. The hydroxyl groups of the sugar and phenol are then deprotected to give etoposide.

Protection of the phenol is commonly accomplished with the carbobenzoxy (CBz) group or other acyl protecting groups.² In recent years, however, Wang^{3a} and Allevi^{3b} have independently reported couplings without protecting the phenol. In those cases, isolation has been difficult, and the reported yields of the desired coupled product are not as high as when the phenol group is protected. Also both Wang and Allevi require the initial use of the protected sugar in the C-1"-β anomeric orientation for the coupling step.

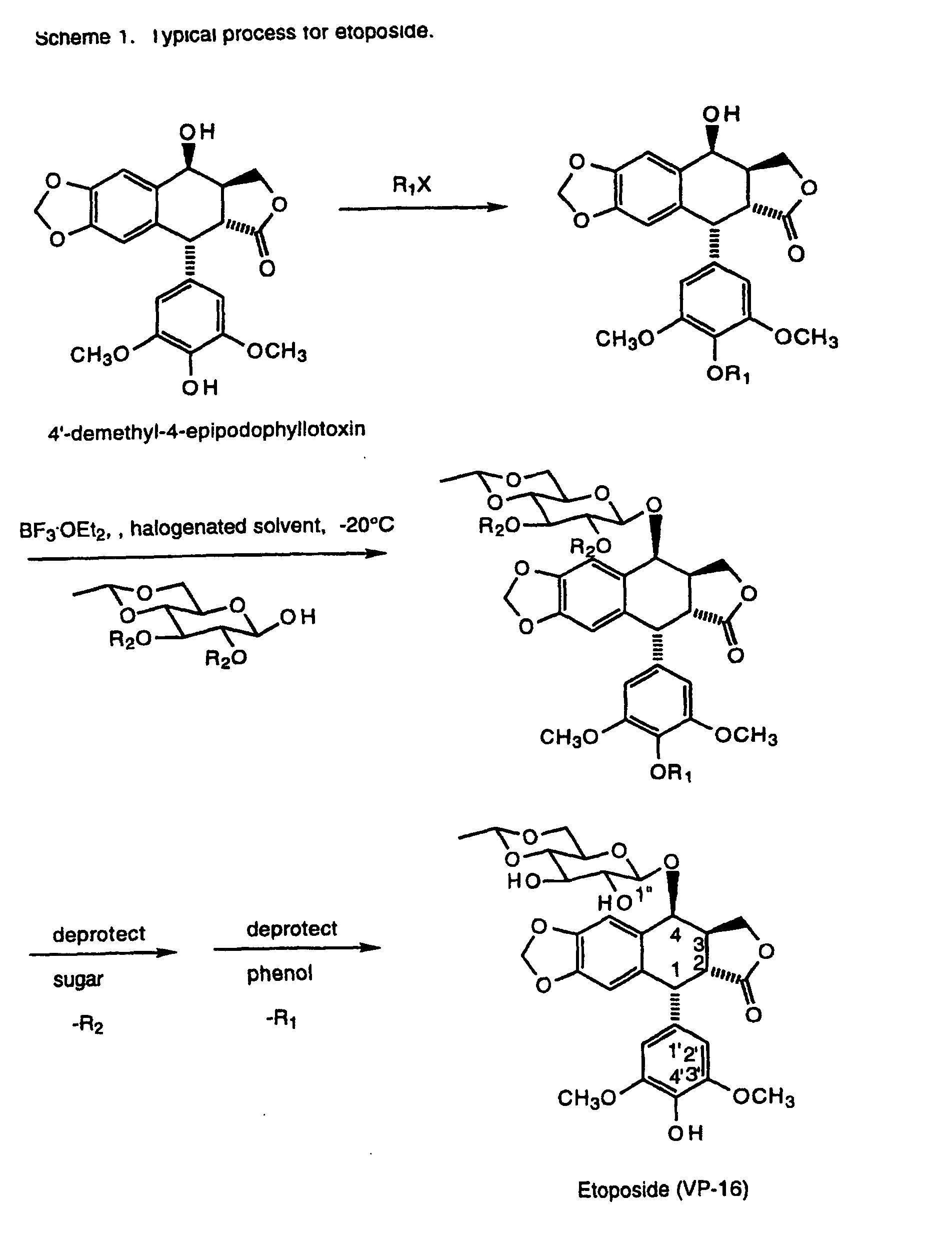

The key step in this process is the coupling reaction. It is generally accomplished by attack of the anomeric oxygen of the sugar on a BF₃·OEt₂ generated C-4 lignan carbocation, which can give a mixture of C-1"-α and -β anomers. To achieve selectivity, prior art teaches making a sugar which is mostly β so the reaction will proceed predominantly with retention of the β-anomeric configuration^{1,2,3}. This has meant the use of 2,3-acyl protected sugars, which can be made predominantly β, however, these compounds suffer from the disadvantage that the protecting groups can be difficult to remove cleanly and usually require the stoichiometric use of heavy metals. The use of ether protecting groups is rare because the β sugars are not readily made (*e.g.* 2,3,4,6-tetra-O-benzyl-β-D-glucopyranose has never been reported). A benzyl ether protecting group offers the advantage of mild, neutral conditions for deprotection.

Ohnuma and Hoshi⁴ reported the coupling of 2,3-di-*O*-benzyl-4,6-*O*-ethylidene-α,β-D-allopyranose with 4'-*O*-CBZ-4'-demethyl-4-epipodophyllotoxin. Allevi has reported the coupling of 1-*O*-β-trimethylsilyl sugars, including 1-*O*-TMS-2,3,4,6-tetra-*O*-benzyl-β-D-glucopyranose in the presence of trimethylsilyl triflate to achieve high selectivity^{3c,d}.

Silverberg *et al.* recently reported the selective coupling of 4,6 ethylidene 2,3-di-*O*-benzylic-α,β-D-glucopyranoses⁵. In the typically used halogenated solvents, anomerization of the sugar under the reaction conditions was very rapid, and as a result the couplings gave anomeric mixtures (the selectivity of which could be affected by substituents on the benzyl group). The use of, preferably, acetonitrile as the solvent slowed the anomerization of the sugar relative to the rate of coupling and allowed control of the C-1" stereochemistry. The preparation of >85% β 2,3-di-*O*-benzyl-4,6-*O*-ethylidene-D-glucopyranose and it's use in the synthesis of etoposide phosphate was disclosed as well.

In contrast to the prior art problems, we describe a new synthesis of etoposide, accomplished in high yield in only two steps by coupling 2,3-di-*O*-benzyl-4,6-*O*-ethylidene-α,β-D-glucopyranose (i.e., a mixture of the α and β anomers) with 4'-demethyl-4-epipodophyllotoxin (i.e. having an unprotected phenol) using BF₃·OEt₂ in an organic solvent, preferably acetonitrile. The novel intermediate products form unexpectedly with good selectivity, and then, due to the presence of BF₃·OEt_{2,} surprisingly and unexpectedly epimerize from α to β, presumably via a reversible reaction driven by precipitation of the β product, giving very high selectivity (>44:1). The input sugar does not need to be high in β-anomeric content to achieve high selectivity in the coupling. The product is easily isolated and purified by recrystallization. Deprotection of the hydroxyl groups, preferably by hydrogenation under mild conditions readily affords etoposide in high yield and purity.

### SUMMARY AND OF THE INVENTION

Accordingly, the present invention is directed to a process for preparing the compound etoposide **4** having the structure 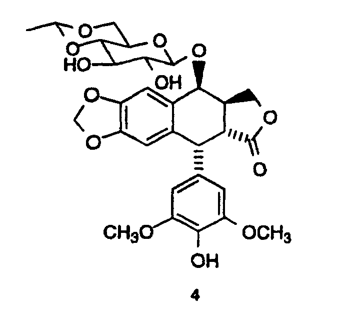 which comprises:
(a) reacting an anomeric mixture of a compound of formula **2** having the structure 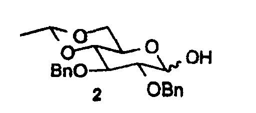 with a compound **1** having the structure 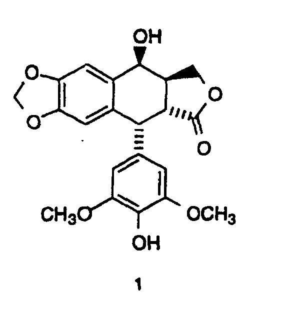 in an organic solvent as a reaction medium and in the presence of a Lewis acid at or below room temperature to form an anomeric mixture of a compound of formula **3** having the structure 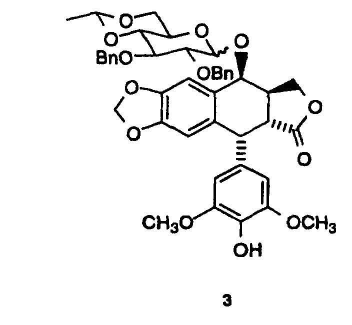
(b) recovering the C-1"-β form of compound **3** substantially free of the α form from said reaction medium; and
(c) removing the hydroxy-protecting groups from the product of step (b).

A preferred embodiment is the aforementioned process wherein said solvent is halogenated or non-halogenated, more preferably, acetonitrile.

Another preferred embodiment is the aforementioned process wherein said hydroxy-protecting groups are removed by hydrogenation at ambient temperature in the presence of a noble metal catalyst.

In another preferred embodiment, the noble metal catalyst is palladium.

In another preferred embodiment, the Lewis acid is boron trifluoride etherate.

In another preferred embodiment, the reaction is carried out at about room temperature to -40°C, more preferably at 0°C to -40°C, and still more preferably at -10°C.

The present invention is also directed to a process of obtaining the C-1"-β anomer of compound **3** comprising forming a solution of an anomeric mixture of compound **3** in an organic solvent, recovering the resulting precipitated product that forms, forming a second solution of said recovered product in an alcohol, crystallizing said C-1"-β anomer from said second solution and recovering crystals of said C-1"-β anomer substantially free of α anomer.

In a preferred embodiment, the alcohol is methanol.

The present invention is also directed to novel intermediate compounds 3 α, β having the formula

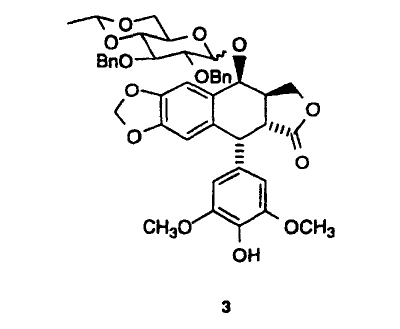

Preferably, the compound **3** is the C-1"-β anomer.

### DETAILED DESCRIPTION OF THE INVENTION

The present invention described herein relates to a novel methodology for the synthesis of etoposide **4** from 4'-demethyl-4-epipodophyllotoxin **1**.

As illustrated for a preferred embodiment (Scheme 2), coupling between **1** and 2,3-di-*O*-benzyl-4,6-*O*-ethylidene-α,β-D-glucopyranose **2**α,β produces a mixture of novel intermediate compounds, **3**β and **3**α. The reaction is allowed to continue, and epimerization of the compounds at C-1" gives very high β:α selectivity. The solid is isolated directly by filtration of the reaction mixture (other workup methods can also be used), and purified to **3**β by recrystallization. **3**β is then hydrogenated to remove the protecting groups, affording etoposide **4**.

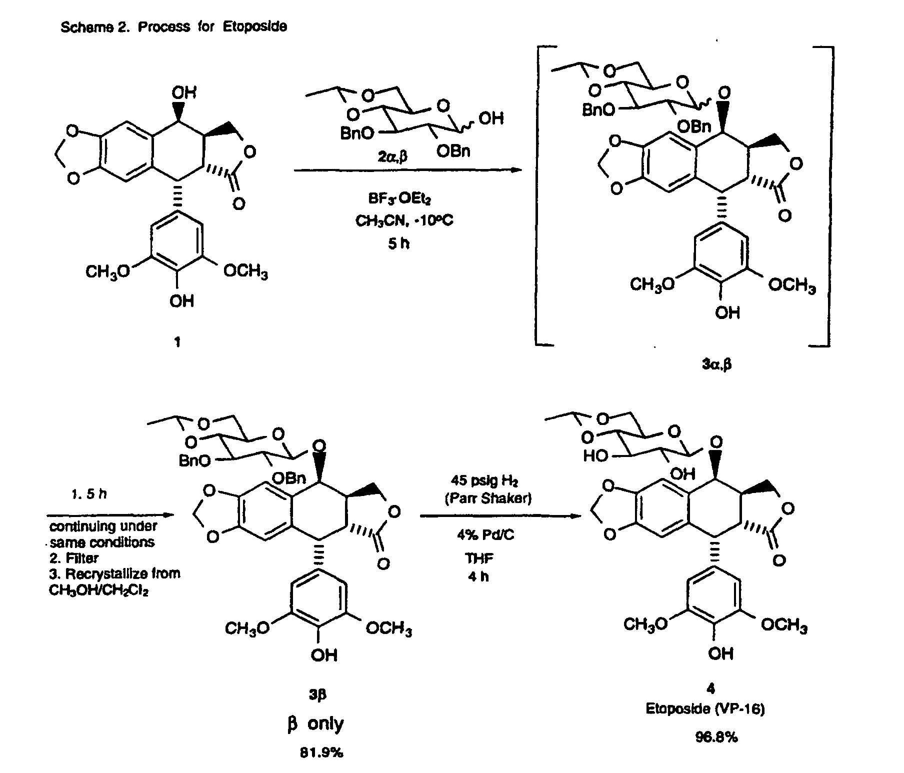

### 1. Coupling

Most of the sugar-lignan couplings (i.e. reaction of compounds **1** and **2**) in the past have been in a very similar vein, essentially no different than the procedure disclosed by Sandoz.¹ Typically, a 4'-protected-4'-demethyl-4-epipododphyllotoxxin is coupled with a 2,3-di-*O*-acyl (ester or carbonate)-protected-β-D-glucopyranose in the presence of boron trifluoride etherate in halogenated solvents (1,2-dichloroethane or DCE, methylene chloride, or chloroform) at -20°C (Scheme 3)¹,²,³. As long as the sugar **2** is entirely of the β configuration at the C-1 position and anomerization under the reaction conditions is slow, the reaction should give almost exclusively the C-1"-β product.

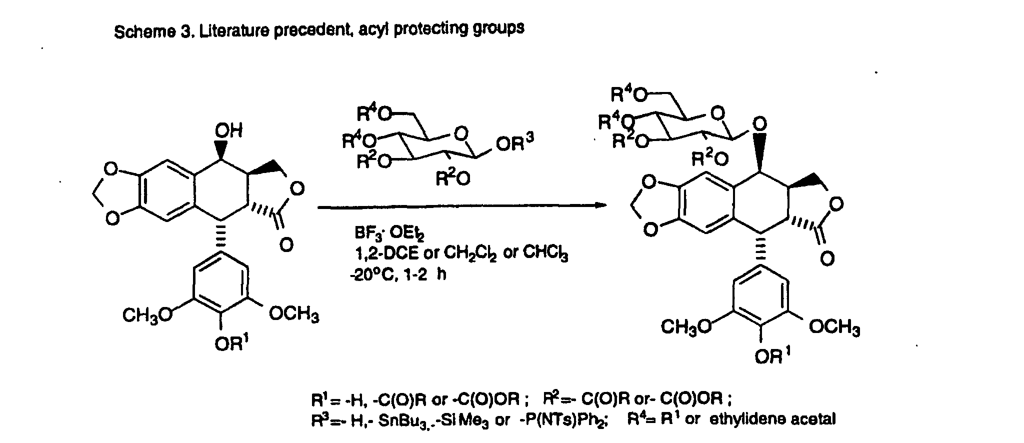

Two departures from the use of acyl protecting groups have been published recently (Scheme 4). Ohnuma and Hoshi⁴ reported the coupling of 2,3-*O*-dibenzyl-4,6-*O*-ethylidene-α,β-D-allopyranose with 4'-*O*-CBZ-4'-demethyl-4-epipodophyllotoxin. Allevi and coworkers^{3c,d} reported that 1-*O*-trimethylsilyl-2,3,4,6-tetra-*O*-benzyl-β-D-glucopyranoside could be coupled with assorted lignans, including **1**, using trimethylsilyl triflate in methylene chloride at -70°C to give almost exclusively the C-1"-β product. The TMS (i.e. trimethylsilyl) group is necessary to get a high percentage of β coupled product and also requires an extra step to incorporate the same into the sugar.

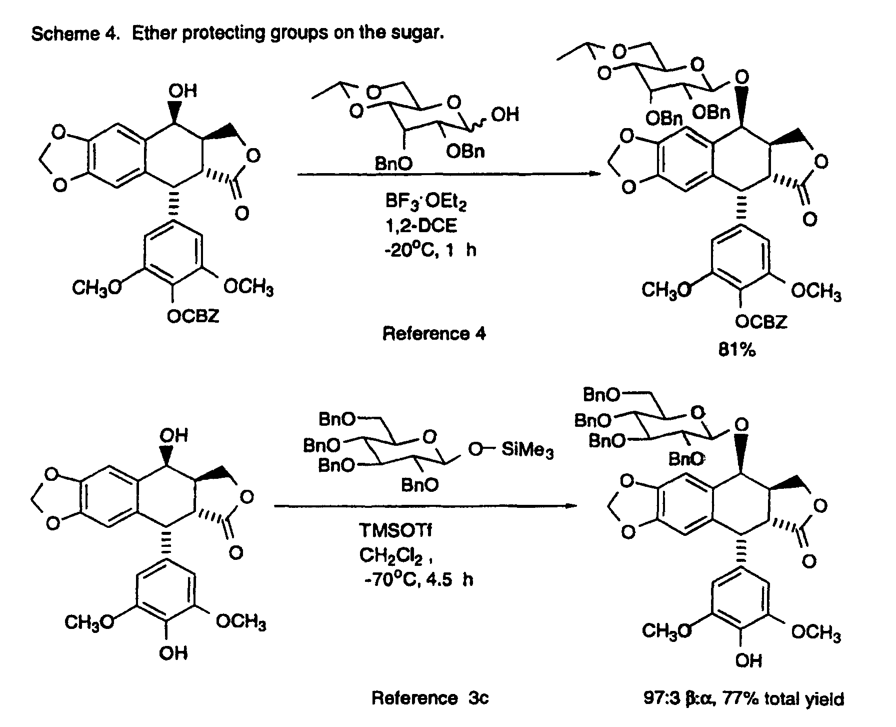

In contrast to the prior art, the process disclosed herein (i.e. Scheme **2**) uses **2** as the sugar or carbohydrate component. The required sugar **2** was prepared by adaptation of known literature methods for analogous compounds.⁴ If desired, the mixture of anomers of **2** may be converted to a mixture that is >85% β. This involves either a) recrystallization from hexanes or heptane; b) allowing the oily mixture of anomers to stand at room temperature for 2 months; or c) recrystallizing from methanol/water and then heating the solid below it's melting point for a period of 1-7 days. However, it has been surprisingly and unexpectedly found that the anomerically pure sugar **2** β is not required in the coupling step used herein. Coupling of **1** and **2**α,β in the presence of a Lewis acid, preferably boron trifluoride etherate, in an organic solvent, which can be halogenated or non-halogenated, preferably acetonitrile, at room temperature to -40°C, preferably 0°C to -40°C, still more preferably at -10°C, is very rapid, going to completion within about 5 hours.

The initial anomeric ratio of products is dependent on the initial anomeric composition of compound **2**. If starting compound **2** is >90% β, the ratio for coupled product **3** at -40(C is about 8:2 β:α. If the input sugar **2** is 63:37, the initial coupled product **3** ratio is 61:39. However, if the reaction is allowed to continue, the β:α ratio of the products unexpectedly increases. If the products remain in solution, a thermodynamic equilibrium will be established between the two products 3 α and 3 β. This is presumably caused by a reversible uncoupling of the sugar, followed by rapid recoupling. In this specific case, however, the product 3 β precipitates preferentially due to lower solubility from the acetonitrile. The product remaining in the solution (presumably mostly α) then reestablishes the equilibrium, driving the reaction towards the β product. This theory was demonstrated in a reaction in which the product did not precipitate rapidly and the ratio of products moved toward the α. When it finally did precipitate, the ratio moved back towards β. Compound **2** that is not high in β content may be used, although the reaction may take more time to reach the higher ratios. Ratios as high as 97.8:2.2 β: α have been reached. Seeding of the solution with pure **3** β also aids in the precipitation of the product thereby lowering the overall reaction time.

Although it is preferred that the 2,3-hydroxy-protecting groups on glucopyranose compound 2 be benzyl (i.e. R₁ is benzyl), it is contemplated that other aryl methyl groups can be used. For example, substituted benzyl that is substituted with one or more selected from the group consisting of C₁-₄ alkyl, hydroxy, phenyl, benzyl, halogen such as fluoro_{,} chloro, bromo and iodo, alkoxy, nitro and carboxylic acids and esters thereof. Suitable substituted benzyl groups include 2-methyl benzyl, 3-methyl benzyl, 4-methyl benzyl, 1 or 2-naphthyl, 2, 3 or 4-phenyl benzyl, 4-methoxy carbonyl benzyl, 2,6-dichlorobenzyl, 2-fluorobenzyl and pentafluorobenzyl.

The glucopyranose may further have the structure of Compound **2a** 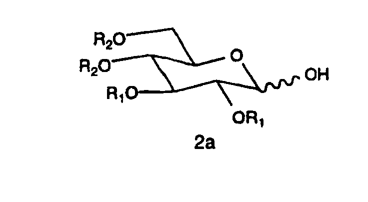 where R₁ is as above and R₂ is the same as R₁, or the two R₂ groups taken together are a C₁₋₅ alkylidene group. Preferably, the two R₂ groups together are ethylidene. In alternative embodiments, the two R₂ groups together may be propylidene or isopropylidene.

Compounds **2** and **2a** are prepared by known procedures such as that described in U.S. Patent No. 4,997,931.

The product 3 can be isolated directly by filtration of the reaction mixture. This yields a crude solid in a weight yield of 95% and a purity of ∼90%. There is a small amount of **3**α. This isolation gives much better results than Wang's or Allevi's methods. The solid **3** is then recrystallized by dissolving in methylene chloride, adding methanol, distilling off most of the methylene chloride, and cooling. **3**β is obtained in 78.6% yield with purity >99%. This yield is higher than Wang's (60%)^{3a} or Allevi's coupling yields and affords higher quality product (77% yield for total of α and β, 97:3 β:α)^{3c}, and is achieved without chromatography. Other isolation methods can also be used to achieve yields as high as 81.9%, as detailed in the experimental section.

The initial ratio of C-1"-β:α in the coupled products is affected by numerous factors, including temperature and solvent.

The Lewis acid used in the coupling step can be for example AlCl₃, ZnCl₂, Et₂AlCl, CF₃SO₃H, CF₃SO₃Ag, Zn(CF₃SO₃)₂, TMSCF₃SO₃ and boron trifluoride etherate. Preferably, it is boron trifluoride etherate.

The organic solvent used as a reaction medium may be halogenated or non-halogenated. Examples are propionitrile, acetone, methylene chloride, chloroform, 1,2-dichloroethane, acetonitrile and mixtures thereof. The preferred solvent for the coupling reaction is acetonitrile since it alows for direct crystallization of the **3**β product therefore allowing for purification to drive the reaction to the desired product.

The coupling reaction is generally carried out at or below room temperature and preferably at about 0°C to -40°C. Preferred temperature is -10°C.

### 2. Deprotection

In most of the previous syntheses of etoposide, the sugar is protected with acyl groups. Since etoposide is sensitive to both acid and base, removal of these is not trivial. Commonly, stoichiometric amounts of heavy metal compounds such as zinc acetate are used to effect the deprotection. This is not desirable in an industrial process. Furthermore, serious degradation is still often observed, depending on the protecting group being removed. For example, Allevi reports only a 70% yield for this step in a recent synthesis of etoposide^{3b}. In etoposide syntheses, the phenol is usually also protected, most commonly with the benzyloxycarbonyl group, and must also be deprotected; recent publications³ have shown that protection of the phenol is not necessary, however.

In the invention described herein, after recovery of the C-1"-β anomer of compound **3**, the hydroxy protecting groups are removed by known methods and preferably by hydrogenation. The hydrogenation deprotection step proceeds efficiently to produce etoposide in high yield with minimal degradation. Compound **3** is very labile and sensitive to both acid and base. Existing processes of using acids or bases to remove the hydroxy protective groups usually result in decomposition of a portion of the desired product. In addition, the deprotection steps can cleave the ethylidene group from the glucopyranose.

The hydrogenation may be accomplished by a number of known processes. Typically, the hydrogenation is done in the presence of a noble metal catalyst in a suitable solvent or solvent mixture.

In preferred embodiments, the hydrogenation is carried out using 4% palladium on carbon in a solution of compound **3** C-1"-β anomer in tetrahydrofuran (THF) or acetone. The mixture is hydrogenated for several hours, typically 3-6 hours, at 40-50 psig hydrogen. The catalyst may then be removed by filtration, and the etoposide recrystallized from THF-water or methanol-water solutions.

In the invention described herein, the protecting groups are preferably removed by hydrogenation over a Pd/C catalyst. A yield of 96.8% has been obtained. There are several advantages compared to previous etoposide syntheses: a) there is only one deprotection step, not two; b) the use of heavy metals such as zinc acetate is avoided in deprotecting the sugar; c) hydrogenation conditions are mild, neutral, and high yielding; d) chromatography is not required to obtain etoposide of high Purity.

To summarize, the process has the following advantages: a) it is a short and efficient synthesis of etoposide without any chromatographic purification; b) the compound **1** phenol during the coupling reaction is unprotected and the sugar protecting groups on resulting compound **3**β are removed readily in high yield (the overall yield for the two steps is 79.3% - Allevi reports 35.7%^{3b} and 70%^{3c} overall, Wang 54%^{3a)}; c) very high β:α ratios of coupled product **3** can be obtained even when starting from a sugar **2** of moderate β:α ratio, in view of the novel epimerization of **3** α to **3β;** and d) it is not necessary to make C-1 derivatives on sugar compound **2** prior to coupling, as is taught by Allevi^{3c,d} (e.g. using 1-*O*-β-trimethylsilyl sugars).

The specific examples that follow illustrate the invention herein and are not meant to limit the scope thereof, variations of which will be evident to one skilled in the art.

### Example 1

### 2i3'-Di-O-benzyletoposide (3β) (direct filtration):

A 250 ml three-necked round-bottomed flask was oven-dried, fitted with a septum and N₂ inlet, mechanical stirrer, and thermometer, and cooled under N₂. Compounds 1 (5.00 g, 12.49 mmol) and **2**α,β (5.792 g, 14.99 mmol, 1.2 eq.) were added, followed by anhydrous acetonitrile (111 ml). The slurry was stirred at room temperature for a few minutes and then cooled to -40°C. Boron trifluoride etherate (4.15 ml, 33.72 mmol, 2.7 eq.) was added by syringe. The slurry turned yellow and the solid began to dissolve, but within eight minutes the slurry appeared thicker as the product was precipitating. HPLC showed 14% **1**, 14.6% **3**α, and 66.2% **3**β. After 25 min., anhydrous acetonitrile (55.5 ml) was added slowly (keeping the temperature below -35°C) to thin the mixture. After 18 h (i.e. hours) at - 40°C, HPLC showed a 97.5:2.5 ratio of **3**β:**3**α. After a total of 19 h, the solid was collected by vacuum filtration of the -40°C reaction mixture. The solid was washed twice with very cold (-40°C to -20°C)) acetonitrile and dried in the vacuum oven. The crude solid was shown to have an area percent of 96. The solid was dissolved in CH₂Cl₂ (54 ml) in a two-necked 500 ml round-bottomed flask with a mechanical stirrer. Methanol (270 ml) was added slowly and the solution stirred for a few minutes, and crystals were noted. The mixture was warmed to reflux and distilled at atmospheric pressure until 55 ml of distillate was collected. The mixture was stirred at room temperature for 2 h, then cooled in ice for 2.5 h. The white crystalline compound was collected by vacuum filtration and washed twice with cold methanol. The solid was dried under vacuum at 40°C overnight. 7.54 g (78.6% yield) of **3**β was obtained with an HPLC area percent of 99.3. ¹H NMR (CDCl₃): δ7.36-7.19 (m, 8H), 6.99 (dd, J=1.9, 7.5 Hz, 2H), 6.82 (s,1H), 6.55 (s, 1H), 6.24 (s, 2H), 5.97 (d, j=1.2 Hz, 1H), 5.89 (d, J=1.2 Hz 1H), 4.90-4.85 (m, 2H), 4.78-4.71 (m, 3H), 4.59-450 (m, 3H), 4.38 (dd, J=9.0, 10.5 Hz, 1H), 4.23-4.14 (m, 1H), 3.75 (s, 6H), 3.65 (t, J= 9.0 Hz, 1H), 3.54 (t, J=10.2 Hz, 1H), 3.45-3.35 (m, 2H), 3.32-3.27 (m, 1H), 3.23 (dd, J=5.2, 14.1 Hz, 1H), 2.92-2.82 (m, 1H), 1.38 (d, J=5.0 Hz, 3H) ¹³C NMR (CDCl₃): δ174.9, 148.7, 147.0, 146.4, 138.5, 137.8, 134.0, 132.5, 130.6, 128.6,128.3, 128.2, 128.1, 127.9, 127.73, 127.66, 110.6, 109.1, 107.8, 102.3,101.5, 99.5, 81.7, 80.9, 75.4, 75.0, 73.5, 68.2, 67.9, 66.0, 59.2, 56.4, 43.7, 41.3, 37.5, 20.4.

### Example 2

### 2',3'-Di-O-benzyletoposide (3β) (Quench with buffer):

A 250 ml three-necked round-bottomed flask was oven-dried, fitted with a septum, mechanical stirrer, thermometer and N₂ inlet and cooled under N₂. Compound **1** (5.00 g) and anhydrous acetonitrile (111 ml) was added. The slurry was stirred and then cooled to -40°C. A sample of **2**α,β (5.792 g, 1.2 eq.) was added. Boron trifluoride etherate (4.15 ml, 2.7 eq.) was added by syringe. The slurry turned yellow and the solid began to dissolve, but within ten minutes the slurry appeared thicker as the product was precipitating. HPLC after 17 min showed 15% **1**, 19% **3**α, and 54% **3**β. The slurry was stirred at -40°C for 18 h, and pH 7 buffer (Fisher, 0.05 M KH₂PO₄, 222 ml) was added. The mixture was allowed to warm to between 0°C and room temperature. The solid was collected by vacuum filtration, washed twice with water and dried under vacuum (40°C). The crude white solid (9.42 g, 98.1%) thus obtained had an area percent of 85.1% by HPLC. The solid was dissolved in CH₂Cl₂ (56.4 ml) and one percent methanol (282 ml) was added slowly while warming and the solution stirred. The mixture was warmed to reflux and distilled at atmospheric pressure until 56 ml distllate was collected. The mixture was allowed to cool to room temperature, then cooled in an ice bath for 2 h. The white crystals were collected by vacuum filtration and washed twice with cold methanol. The solid was dried in the vacuum oven (40°C, house vacuum) overnight. 7.06 g (73.6% yield) of **3**β was obtained with a 99.1. The area percent was 99.1 as determined by HPLC.

### Example 3

### 2',3'-Di-O-benzyletoposide (3β) (Quench with N-methylmorpholine and toluene extraction):

A 25 ml two-necked round-bottomed flask with a stir bar was oven-dried, fitted with a septum, and N₂ inlet and cooled under N₂. Compounds **1** (0.180 g) and **2**α,β (0.21 g, 1.2 eq.) were added following anhydrous acetonitrile (4 ml). The slurry was stirred and then cooled to -40°C. Boron trifluoride etherate (0.15 ml, 2.7 eq.) was added by syringe. The slurry turned yellow and the solid began to dissolve, but within twelve minutes the slurry looked thicker as the product was precipitating. After 12 min., anydrous acetonitrile (2 ml) was added to enhance stirring. The slurry was stirred at -40°C overnight. N-methylmorpholine (0.45 ml, 9 eq.) was added to quench the reaction. The mixture was diluted with toluene (the solid dissolved) and washed twice with water. The solvent was removed *in vacuo.* The resulting yellow solid was dissolved in hot toluene (2 ml). Methanol (10 ml) was added slowly while keeping the solution warm and the resulting solution was stirred. The mixture was allowed to cool to room temperature and when the temperature approached ambient, the product began to crystallize. The slurry was cooled in an ice bath for 2.5 h. The white crystals were collected by vacuum filtration and washed twice with cold methanol. The solid was dried in vacuo (40°C) overnight. 0.18 g (51.3% yield) of **3**β was obtained with a 99.1 area percent.

### Example 4

### 2',3'-Di-O-benzyletoposide (3β) (Buffer quench and toluene extraction):

A 15 ml round-bottomed flask equipped with a stir bar was oven-dried, fitted with a septum, and cooled under N₂. Compounds 1 (0.18g) and **2**α,β (0.21 g) were added. Anhydrous acetonitrile (4 ml) was added and the slurry was cooled to -40°C. Boron trifluoride etherate (0.15 ml) was added. After 25 min, acetonitrile was added (2 ml). The mixture was stirred overnight at -40°C. pH 7 buffer (0.05 M KH₂PO₄, 8 ml) was added and the mixture warmed to room temperature while stirring then extracted with toluene. The organic phase was washed once with water and concentrated *in vacuo* to an oil. Toluene (1.5 ml) was added and the solution was warmed to 55°C. Methanol (7.5 ml) was added and the solution was cooled while stirring to room temperature and then 0°C. The solid was collected on a Buchner funnel, washed twice with cold methanol, and dried in the vacuum oven (40°C, house vacuum) overnight. This produced 0.1830 g (53.0%) of **3**β with an HPLC percent of 99.3.

### Example 5

### 2', 3'-di-0-benzyletonoside (3β):

A 500 ml three-necked round-bottomed flask was oven-dried, fitted with a mechanical stirrer, thermometer and N₂ inlet, and cooled under N₂. Compounds 1 (9.0g, 22.5 mmol) and **2** α,β (10.53 g, 27.2 mmol) were added. Anyhydrous acetonitrile (200 ml) was added. The slurry was stirred and cooled to -13 °C. Boron trifluoride etherate (4.45 ml, 33.7 mmol) was added in one portion. The solution was stirred at -10 °C for 5 hours, at which time HPLC showed a ratio of >95:5 β: α. Crystals began precipitating after 10-15 min. Pyridine (8:22 ml, 102 mmol) was added and stirred for 10 min. The mixture was concentrated *in vacuo* to a volume of 70 ml. Dichloromethane (100 ml) was added and the solution was washed three times with water (100 ml each). The organic layer was concentrated to 70 ml and acetonitrile (100 ml) was added. The solution was concentrated to 80 ml. Addition of acetonitrile and concentration was repeated. The solution was heated to 65-70 °C and then cooled slowly to 20 °C and stirred for one hour. The slurry was cooled to 0 °C and stirred for two hours. The product was collected by vacuum filtration and washed with cold acetonitrile (50 ml). The wet cake was dissolved in dichloromethane (150 ml) and then concentrated to 100 ml. Methanol (300 ml) was added and the dichloromethane was distilled off at atmospheric pressure. The volume was maintained at 325 ml by addition of methanol. The slurry was cooled to 20 °C and stirred for one hour and then cooled to 0 °C and stirred for two hours. The product was collected and washed with cold methanol. The solid was dried in the vacuum oven at 45 °C to yield 14.08 g (81.9% yield) of **3** β.

### Example 6

### Etoposide(4):

THF:CH₃OH (1:1, 30 ml) was added to a mixture of **3**β (2 g) and 4% wet Pd/C catalyst in a Parr-shaker bottle. It was purged three times with hydrogen and hydrogenated at 50 psi. After 3.5 h at room temperature, the slurry was filtered through celite and was washed with warm methanol (56°C, 3 x 15 ml). The combined filtrates were evaporated to a solid to which water (25 ml) was added and evaporated to remove ∼5 ml. The resulting aqueous slurry (∼25 ml) was stirred at room temperature for 15 min and at 20°C for 15 min, and filtered. The solid etoposide was washed with water (2 x 5 ml) and dried at 40°C to constant weight (1.42 g, 92.5% yield). According to HPLC, it contained 99.7% of **4**.

### Example 7

### Etoposide (4):

A solution of **3** β (20 g, 26.01 mm01) in THF (150 ml) was added to a mixture of wet 5%-Pd/C (2.5 g, 50% water wet) in THF (50 ml) in a Buchi apparatus under nitrogen atmosphere. It was purged three times with nitrogen and hydrogen, and hydrogenated at 50 psi at room temperature. After 4 h, it was carefully depressurized and transferred out from the hydrogenation setup, which was washed with warm THF (55 °C, 2 x 200 ml). The rich reaction mixture was filtered on a Buchner funnel containing a 0.45 µm nylon membrane filter and celite-521. The celite cake was washed with the THF which had been used to clean the reactor. The filtrate was evaporated to ∼50 ml of a light yellow solution. Water (300 ml) and 10% NaHSO₃ (3 ml) were added and stirred at room temperature for 30 min and at 3 °C for 30 min. The resulting colorless slurry was filtered, washed with cold water (2 x 25 ml) and dried to give etoposide (**4**, 14.83 g) in 96.8% weight yield with purity of 100.0%.

### Example 8

### Etoposide (4):

A slurry of **3**β (25 g, 32.5 mmol) in acetone (250 ml) and 5% Pd/C (50% water wet) was charged to a 1L hydrogenating vessel. Additional amount (250 ml) acetone was used to wash in the **3**β and the catalyst. The resulting slurry was purged six times with nitrogen followed by hydrogen, and hydrogenated at 3 bar pressure and room temperature for an hour. On completion of the reaction, MeOH (250 ml) was added to solubilize the product, and the catalyst was removed by filtration. The reactor was rinsed with MeOH (250 ml), which was subsequently used in washing the spent catalyst. The combined filtrates were treated with NaHSO₃ solution (1N, 7.5 ml) to remove any color and the solvent exchanged for MeOH by distillative exchange. The resultant MeOH slurry (325 ml) was heated to 65 °C, at which point water (500 m l) was added. The crystalline slurry was heated at 85 °C to form a clear solution, seeded with etoposide and cooled slowly to room temperature and then to 0 °C where it was held for an hour. The product was isolated by filtration and dried *in vacuo* to give etoposide (**4**, 17.6-17.99 g) in an average of 93.3% weight yield with HPLC area % greater than 99% based on three runs.

### REFERENCES

1.
   a) von Kuhn, M.; von Wartburg, A. *Helv. Chim. Acta* **1968**, *51*, 1631.
   b) Kuhn, M.; Keller-Juslen, C. Renz, J.; von Wartburg, A. *Canadian Patent No. 956939,* Oct. 24, 1974.
2.
   c) Keller-Juslen, C.; Kuhn, M.; von Wartburg, A. *J. Med. Chem.* **1971**, *14*, 936.
   d) Keller-Juslen, C.; Kuhn, M.; Renz, J.; von Wartburg, A. *U.S. Pat. 3,524,844,* 1970.
   e) Kuhn, M.; von Wartburg, A. *Helv. Chim. Acta* **1969**, *52*, 948.
   f) Robin, J-P; Houlbert, N.; Lenain, V. *Eur. Pat. 435709-A1,* 1991.
   g) Robin, J-P; Lenain, V. *Eur. Pat. 445021-A2,*1991.
   h) Saito, H.; Nishimura, Y.; Kondo, S.; Umezawa, H. *Chem. Lett.* **1987**, 799.
   i) Kolar, C. *Eur. Pat. 394907-A1,* 1990.
   j) Kolar, C.; Moldenhauer, H.; Kneissl, G. J. *Carbohyd. Chem.* **1990**, *9*, 571.
   k) Sterling, J.; Nudelman, A.; Herzig, J.; Keinan, E.; Weiner, B.Z. U.S. *Pat 4,900,824,* 1990.
   l) Nudelman, A.; Herzig, J.; Keinan, E.; Weiner, B.Z.; Sterling, J. *Eur. Pat. 226202*,1987.
   m) Hashimoto, S.; Honda, T.; Ikegami, S. *Tetrahedron Lett.* 1991, *32*, 1653.
   n) Kurabayash, K.; Kinoshita, H.; Saito, H.; Takahashi, T. *Eur. Pat., 111058-A1*, 1984.
   o) Fujii, T.; Chikui, Y. *U.S. Pat. 4,757,138,* 1988.
   p) *Japanese Kokai* J58-225-096.
3.
   a) Wang, Z.; Ma, M.; Zhang, C. *U.S. Patent 5,206,350,* Apr. 27, 1993.
   b) Allevi, P.; Anastasia, M.; Ciuffreda, P.; Sanvito, A.M.; Macdonald, P. *Tet. Lett.***1992**, *33, 4831.*
   c) Allevi, P.; Anastasia, M.; Ciuffreda, P.; Bigatti, E.; Macdonald, P. J. *Org. Chem.* **1993**, *58,* 4175*.*
   d) Allevi, P.; Anastasia, M.; Bigatti, E.; Macdonald, P. *PCT Patent Application No.* WO *93*/*02094.,* Feb. 4, 1993.
4. Ohnuma, T.; Hoshi, H. *U.S. Patent No. 4,997,931,* Mar. 5, 1991.
5. Silverberg, L.J.; Vemishetti, P.; Dillon, J.L., Usher, J.J.; *U.S. Patent 5,459,248,* Oct. 17, 1995.

## Claims

1. A process for preparing the compound etoposide **4** having the structure 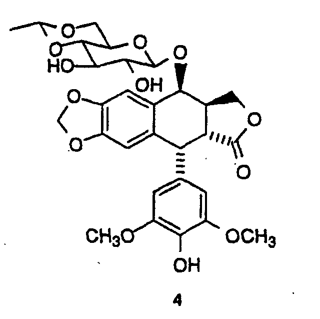 which comprises:
(a) reacting an anomeric mixture of a compound of formula **2** having the structure 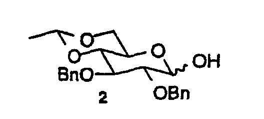 with a compound **1** having the structure 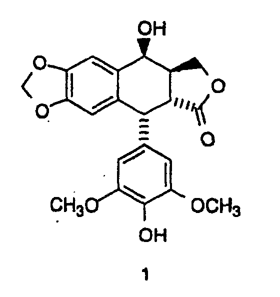 in an organic solvent as a reaction medium and in the presence of a Lewis acid at or below room temperature to form an anomeric mixture of a compound of formula **3** having the structure 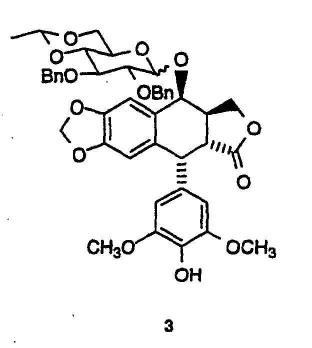
(b) recovering the C-1"-β form of compound **3** substantially free of the α form from said reaction medium; and
(c) removing the hydroxy-protecting groups from the product of step (b).

2. The process of claim 1 wherein said solvent is a halogenated or non-halogenated solvent.

3. The process of claim 2 wherein said solvent is acetonitrile.

4. The process of claim 1 wherein said hydroxy protecting groups are removed by hydrogenating at ambient temperature in the presence of a noble metal catalyst.

5. The process of claim 4 wherein said noble metal catalyst is palladium.

6. The process of claim 1 wherein said Lewis acid is boron trifluoride etherate.

7. The process of claim 1 wherein said reaction is carried out at about room temperature to -40°C.

8. The process of claim 7 wherein said reaction is carried out at a temperature of about 0°C to -40°C.

9. The process of claim 8 wherein said reaction temperature is -10°C.

10. A process of obtaining the C-1"-β anomer of compound **3** comprising forming a solution of an anomeric mixture of compound **3** in an organic solvent, recovering the resulting precipitated product that forms, forming a second solution of said recovered product in an alcohol, crystallizing said C-1"-β anomer from said second solution and recovering crystals of said C-1"-β anomer substantially free of α anomer.

11. The process of claim 10 wherein said alcohol is methanol.

12. A compound **3** having the structure

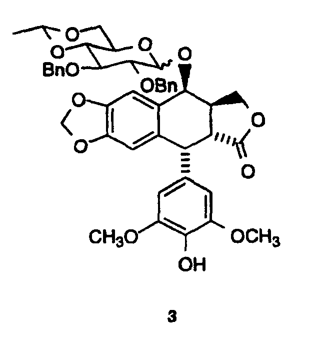

13. The compound of claim 12 wherein said compound **3** is the C-1"-β anomer.

## Patentansprüche

1. Verfahren zur Herstellung der Verbindung Etoposid **4** mit der Struktur 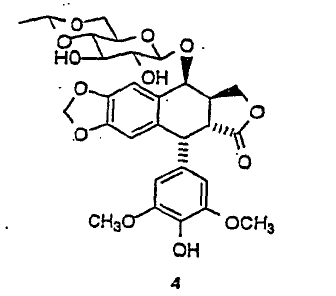 welches umfasst:
(a) Umsetzen eines Anomerengemisches einer Verbindung der Formel **2** mit der Struktur: . 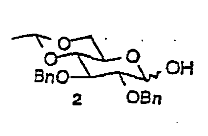 mit einer Verbindung 1 mit der Struktur: 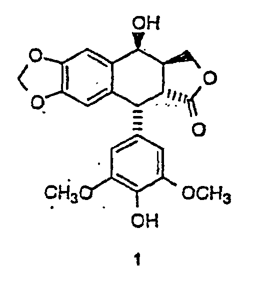 in einem organischen Lösungsmittel als Reaktionsmedium und in Anwesenheit einer Lewis-Säure bei oder unterhalb von Raumtemperatur zur Bildung eines Anomerengemisches einer Verbindung der Formel **3** mit der Struktur: 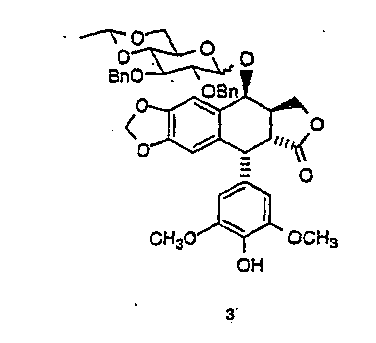
(b) Gewinnen der C-1 "-β-Form der Verbindung 3, die im wesentlichen frei von der α-Form ist, aus dem Reaktionsmedium; und
(c) Entfernen der Hydroxy-Schutzgruppen von dem Produkt aus Schritt (b).

2. Verfahren nach Anspruch 1, wobei das Lösungsmittel ein halogeniertes oder nicht-halogeniertes Lösungsmittel ist.

3. Verfahren nach Anspruch 2, wobei das Lösungsmittel Acetonitril ist.

4. Verfahren nach Anspruch 1, wobei die Hydroxy-Schutzgruppen durch Hydrieren bei Raumtemperatur in Anwesenheit eines Edelmetall-Katalysators entfernt werden.

5. Verfahren nach Anspruch 4, wobei der Edelmetall-Katalysator Palladium ist.

6. Verfahren nach Anspruch 1, wobei die Lewis-Säure Borontrifluoridetherat ist.

7. Verfahren nach Anspruch 1, wobei die Reaktion bei etwa Raumtemperatur bis -40C° durchgeführt wird.

8. Verfahren nach Anspruch 7, wobei die Reaktion bei einer Temperatur von etwa OC° bis -40C° durchgeführt wird.

9. Verfahren nach Anspruch 8, wobei die Reaktionstemperatur -10°C ist.

10. Verfahren zum Erhalt des C-1"-β-Anomeren der Verbindung **3**, umfassend das Erzeugen einer Lösung eines Anomerengemisches der Verbindung **3** in einem organischen Lösungsmittel, Gewinnen des sich bildenden resultierenden ausgefällten Produkts, Erzeugen einer zweiten Lösung des gewonnenen Produkts in einem Alkohol, Kristallisieren des C-1"-β-Anomeren aus der zweiten Lösung und Gewinnen von Kristallen des C-1"-β-Anomeren, die im wesentlichen frei von α-Anomeren sind.

11. Verfahren nach Anspruch 10, wobei der Alkohol Methanol ist.

12. Verbindung **3** mit der Struktur

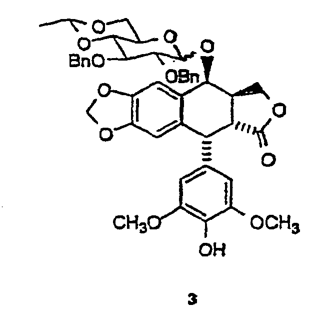

13. Verbindung nach Anspruch 12, wobei die Verbindung **3** das C-1 "-β-Anomer ist.

## Revendications

1. Procédé de préparation du composé 4 étoposide ayant la structure . 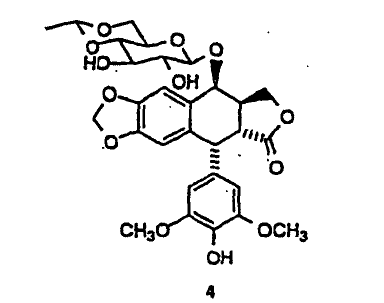 qui consiste à :
(a) faire réagir un mélange anomérique d'un composé de formule 2 ayant la structure . 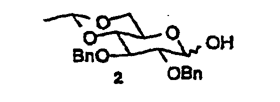 avec un composé 1 ayant la structure : 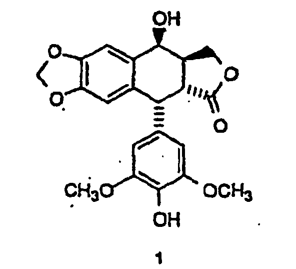 dans un solvant organique à titre de milieu de réaction et en présence d'un acide de Lewis à la température ambiante ou à une température inférieure pour former un mélange anomérique d'un composé de formule 3 ayant la structure . 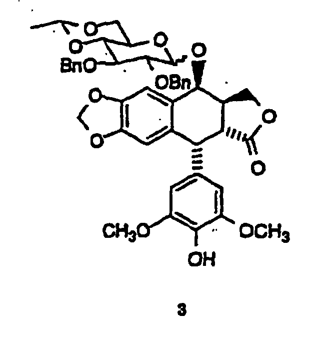
(b) récupérer la forme C-1"-β du composé 3 sensiblement exempt de la forme α à partir dudit milieu de réaction ; et
(c) éliminer les groupements de protection du groupement hydroxy du produit de l'étape (b).

2. Procédé selon la revendication 1, dans lequel ledit solvant est un solvant halogéné ou non halogéné.

3. Procédé selon la revendication 2, dans lequel ledit solvant est l'acétonitrile.

4. Procédé selon la revendication 1, dans lequel lesdits groupements de protection du groupement hydroxy sont éliminés par hydrogénation à la température ambiante en présence d'un catalyseur à base de métal noble.

5. Procédé selon la revendication 4, dans lequel ledit catalyseur à base de métal noble est le palladium.

6. Procédé selon la revendication 1, dans lequel Ledit acide de Lewis est du trifluorure de bore de l'éthérate.

7. Procédé selon la revendication 1, dans lequel ladite réaction est réalisée à la température ambiante, à -40°C environ.

8. Procédé selon la revendication 7, dans lequel ladite réaction est réalisée à une température d'environ 0°C à -40°C.

9. Procédé selon la revendication 8, dans lequel ladite température de réaction est de -10°C.

10. Procédé permettant d'obtenir l'anomère C-1"-β du composé 3 consistant à préparer une solution d'un mélange anomérique du composé 3 dans un solvant organique, récupérer le produit précipité résultant qui se forme, préparer une seconde solution dudit produit récupéré dans un alcool, faire cristalliser ledit anomère C-1"-β dans ladite seconde solution et récupérer les cristaux dudit anomère C-1"-β sensiblement exempt de l'anomère α.

11. Procédé selon la revendication 10, dans lequel ledit alcool est le méthanol.

12. Composé 3 ayant la structure .

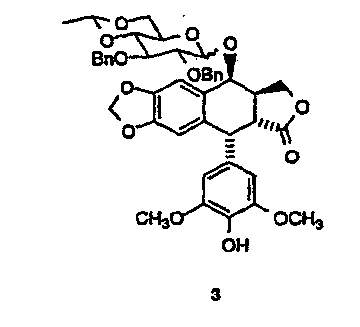

13. Composé selon la revendication 12, dans lequel ledit composé 3 est l'anomère C-1"-β.
